Europäisches Patentamt

European Patent Office

Office européen des brevets

(19)

(11) Numéro de publication : **0 445 150 B1**

(12) **FASCICULE DE BREVET EUROPEEN**

(45) Date de publication du fascicule du brevet :
30.09.92 Bulletin 92/40

(51) Int. Cl.⁵ : **A61F 6/14**

(21) Numéro de dépôt : **89912844.1**

(22) Date de dépôt : **22.11.89**

(86) Numéro de dépôt international :
**PCT/BE89/00049**

(87) Numéro de publication internationale :
**WO 90/05507 31.05.90 Gazette 90/12**

(54) **DISPOSITIF INTRA-UTERIN.**

(30) Priorité : **23.11.88 BE 8801324**

(43) Date de publication de la demande :
**11.09.91 Bulletin 91/37**

(45) Mention de la délivrance du brevet :
**30.09.92 Bulletin 92/40**

(84) Etats contractants désignés :
**AT BE CH DE ES FR GB IT LI LU NL SE**

(56) Documents cités :
**EP-A- 0 100 924**
**EP-A- 0 160 633**
**EP-A- 0 191 747**

(56) Documents cités :
**GB-A- 1 129 712**
**US-A- 3 905 360**
**US-A- 3 954 103**
**US-A- 3 957 042**

(73) Titulaire : **Wildemeersch, Dirk**
**Vossenhul 8**
**B-8300 Knokke-Heist (BE)**

(72) Inventeur : **Wildemeersch, Dirk**
**Vossenhul 8**
**B-8300 Knokke-Heist (BE)**

(74) Mandataire : **Prignot, Jean et al**
**OFFICE HANSSENS SPRL Square**
**Marie-Louise, 40 - bte 19**
**B-1040 Bruxelles (BE)**

**Description**

La présente invention a pour objet un dispositif intra-utérin perfectionné, qui présente une grande efficacité d'action tout en étant remarquablement bien toléré dans la matrice.

Dans le domaine des dispositifs intra-utérins, notamment des stérilets, diverses solutions ont été proposées en vue de réduire l'inconfort que causent les supports de la matière active, ainsi que les risques qu'ils entraînent de blessure de la matrice. Une de ces solutions a été décrite dans la publication EP-A-0100924 au nom de Bauer, qui propose un dispositif intra-utérin constitué d'un ancrage à la paroi de la matrice, en forme de vis ou de grappin, solidaire d'une chaînette de cuivre constituant la matière active, chaînette qui se présente sous forme d'un brin unique, ou encore d'une boucle fermée sur elle-même. Une autre solution a été proposée dans la publication BE-A-901.652, au nom du demandeur de la présente demande, suivant laquelle le stérilet est constitué d'éléments creux en matière active dans la cavité le la matrice, percés de part en part et disposés bout à bout pour former un canal longitudinal permettant le passage d'une aiguille, ces éléments étant réunis en un assemblage non rigide, cet assemblage étant solidaire d'un fil pourvu d'un dispositif d'accrochage au tissu de la matrice, convenant pour l'insertion à l'aide d'une aiguille.

Les stérilets décrits dans ces deux publications présentent sur les stérilets conventionnels, solidaires de support rigides, l'avantage d'une part d'être librement déformables, et ainsi de suivre les mouvements de la matrice sans imposer à cette dernière des contraintes susceptibles de causer des douleurs ou même des blessures, et d'autre part de permettre, en liaison avec les déformations du stérilet engendrées par les mouvements de la matrice, un déplacement des éléments actifs de ce dernier dans la cavité de la matrice, et ainsi de répartir physiquement la zone de libération du produit actif dans la cavité de la matrice sur une certaine aire.

L'invention a pour objet de fournir un dispositif intra-utérin permettant de tirer profit de ces mouvements de la matrice pour encore accroître la zone de libération du produit actif dans la cavité de la matrice.

En effet, dans les dispositifs de l'art antérieur, l'aire de libération de la substance active est limitée d'une part du fait que, dans un brin, ou même dans une boucle unique, une action exercée sur une partie du brin ou de la boucle en vue d'assurer son déplacement, est susceptible d'avoir une répercussion sur les autres parties du brin ou de la boucle, et donc de restreindre leur aire de déplacement, et d'autre part du fait que la substance active est libérée à partir d'éléments disposés à la suite les uns des autres pour fo±mer une chaîne en fo±me de brin ou de boucle, l'aire d'action de chaque maillon de la chaîne étant limitée par l'assujettissement de ce maillon aux maillons adjacents.

La présente invention a pour objet un dispositif intra-utérin du type décrit dans les deux publications cidessus, c'est-à-dire constitué d'un élément déformable, en une matière active dans la cavité de la matrice, solidaire d'un dispositif de fixation à la paroi de la matrice et plus particulièrement du type décrit dans BE-A-901652, dans lequel le dispositif de fixation à la paroi de la matrice est constitué d'un fil pourvu d'un dispositif d'accrochage à la paroi de la matrice, convenant pour l'insertion à l'aide d'une aiguille. Plus particulièrement, l'invention a pour objet un dispositif intra-utérin du type précité qui présente par rapport aux dispositifs intra-utérins connus l'avantage d'un accroissement important de l'aire de libération de l'élément actif dans l'ensemble de la cavité dans laquelle il se trouve, cet accroissement résultant d'un déplacement aléatoire individuel de chacun des éléments constituant le dispositif sous l'effet des mouvements de la matrice.

Ce but est atteint, suivant une caractéristique de l'invention, en utilisant comme élément déformable en une matière active dans la cavité de la matrice, une fibre en matière plastique libérant une substance active dans la cavité de la matrice, et en formant cette fibre en plusieurs brins ou boucles juxtaposés et maintenus ensemble en une même zone d'assemblage de dimensions réduites par rapport à la longueur des brins ou des boucles, pour que les brins ou boucles, en-dehors de cette zone d'assemblage, puissent librement se déplacer dans la cavité de la matrice, indépendamment les uns des autres.

Des fibres convenables pour la mise en oeuvre de l'invention sont par exemple des fibres de polymères perméables aux stéroides, du type de celles qui ont été developpées ces dernières années. Ces fibres libèrent des substances actives sur le plan d'une action anticonceptionnelle, ou encore sur le plan d'une action thérapeutique, et ont jusqu'à présent été utilisées enroulées de manière conventionnelle sur des supports intra-utérins en T ou en 7, à la manière des fils de cuivre des dispositifs anticonceptionnels.

Suivant une autre caractéristique de l'invention, le fil pourvu d'un dispositif d'accrochage au tissu de la matrice est rendu solidaire de la fibre dans la zone d'assemblage des éléments de cette dernière.

Suivant encore une autre caractéristique de l'invention, la fibre est enroulée pour former au moins une boucle et deux brins de longueur approximativement égale à celle de la boucle, et l'assemblage de la boucle et des brins est réalisé par un noeud formé dans le fil pourvu du dispositif d'accrochage au tissu de la matrice, ce noeud réalisant ainsi à la fois l'assemblage des divers éléments de la fibre et la fixation de la fibre au fil pourvu du dispositif d'accrochage au tissu de la matrice.

Suivant une autre caractéristique de l'invention, la fibre est formée en plusieurs brins ou boucles, ces brins ou boucles sont chacun rendus solidaires d'un même élément d'assemblage, et le fil pourvu du dispositif d'accrochage au tissu de la matrice est également rendu solidaire de cet élément d'assemblage.

Suivant encore une autre caractéristique de l'invention, l'élément d'assemblage est un élément d'assemblage en forme d'anneau, libérant un espace intérieur suffisant pour permettre le passage d'une aiguille.

L'invention sera mieux comprise en se reportant à la description en même temps qu'au dessin annexé qui représente, uniquement à titre d'exemple, divers modes de réalisation de l'invention, et dans lequel:

– la fig. 1 est une vue en élévation d'un mode de réalisation de l'invention,

– la fig. 2 est une vue en perspective d'un autre mode de réalisation de l'invention.

– la fig. 3 est une vue en élévation d'encore un autre mode de réalisation de l'invention, et

– la fig. 4 est une vue en élévation d'encore un autre mode de réalisation de l'invention, similaire à celui de la fig. 3 sauf en ce qui concerne l'emplacement où le fil pourvu d'un dispositif d'accrochage au tissu de la matrice est rendu solidaire de la fibre.

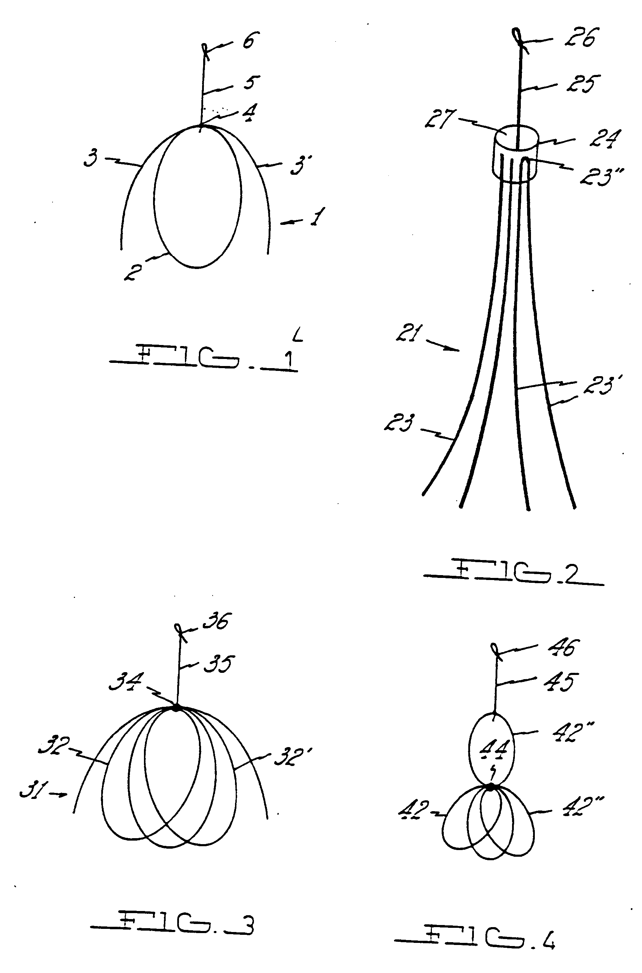

En se reportant à la fig. 1, qui représente un mode de réalisation extrêmement simple de l'invention, le dispositif intra-utérin est constitué d'une fibre 1 formée pour constituer une boucle 2 et deux brins 3,3'. Au point de croisement de la boucle 2 et des brins 3,3', on assure l'assemblage des éléments de la fibre en réalisant autour de ces éléments un noeud 4 à l'aide d'un fil 5, dont l'autre extrémité comporte également un noeud 6 constituant dispositif d'accrochage au tissu de la matrice, convenant pour l'insertion à l'aide d'une aiguille. Ainsi le noeud 4 détermine une zone d'assemblage de dimensions réduites.

Suivant le mode de réalisation de la fig. 2, la fibre 21 se présente sous forme de brins 23,23', solidaires d'un élément d'assemblage 24 en forme d'anneau, élément d'assemblage auquel est également fixé un fil 25 dont l'autre extrémité comporte une déformation 26 constituant dispositif d'accrochage au tissu de la matrice. L'espace intérieur 27 de l'anneau 24 est de dimensions suffisantes pour permettre le passage de l'aiguille destinée à assurer l'insertion du dispositif d'accrochage 26 dans le tissu de la matrice. Les brins solidaires de l'élément d'assemblage 24 peuvent être soit des brins simples comme le brin 23, soit brins 23' pliés à leur partie centrale 23" rendue solidaire de l'élément d'assemblage 24. Dans ce mode de réalisation également, l'élément d'assemblage 24 détermine une zone d'assemblage de dimensions réduites.

En se reportant au mode de réalisation de la fig. 3, la fibre 31 est formée en boucles 32,32' rentenues ensemble par un élément d'assemblage 34 tel que par exemple un point de colle, le fil 35 étant également pris dans cet élément d'assemblage et se terminant à son autre extrémité par un noeud 36 constituant élément d'accrochage au tissu de la matrice, convenant pour l'insertion à l'aide d'une aiguille.

Enfin, le mode de réalisation illustré à la fig. 4 est quasi identique à celui de la fig. 3. La fibre 41 est constituée en boucles 42, 42' maitenues ensemble par un élément d'assemblage 44. A la différence du mode de réalisation de la fig. 3, le fil 45, terminé par un noeud 46 constituant dispositif d'accrochage au tissu de la matrice, est fixé à une boucle 42" de la fibre, et non à l'élément d'assemblage 44.

Dans ces deux derniers modes de réalisation, l'élément d'assemblage détermine également une zone d'assemblage de dimensions réduites.

Suivant les différents modes de réalisation décrits ci-dessus, les divers éléments de la fibre, du fait qu'ils sont assemblés l'un à l'autre dans une seule zone de dimensions réduites, sont libres, en-dehors de cette zone, de se déplacer individuellement dans la matrice sous l'influence des mouvements de celle-ci, assurant ainsi une meilleure répartition dans la matrice de la matière active libérée par la fibre. Il va de soi qu'un même dispositif intra-utérin peut combiner différentes fibres, libérant des matières actives différentes.

L'avantage d'un dispositif intra-utérin conforme à l'invention est qu'il convient à la fois pour couvrir un espace relativement large par rapport à la dimension des fibres, tel que par exemple l'ensemble de la cavité de la matrice, et qu'il peut se disposer sans gêne dans un espace notablement plus étroit tel que le col de l'utérus, tandis que son dispositif de fixation permet de choisir à volonté l'enplacement de fixation le plus adéquat pour le traitement souhaité. C'est ainsi que le dispositif de fixation peut être fixé soit dans la paroi du fond de la matrice, la fibre exerçant alors son action dans la cavité de la matrice, soit à l'extrémité du col de l'utérus, à l'entrée de la matrice, la fibre exerçant alors son action dans le canal de l'utérus.

La diversité des hormones susceptibles d'être libérées par les fibres en matières polymères, oestradiols et progestérones, fait du dispositif de l'invention un instrument particulièrement efficace dans diverses applications. Le dispositif est d'abord efficace comme dispositif anticonceptionnel comportant des fibres libérant des progestagènes, en libérant ces progestagènes soit de manière parfaitement répartie dans la cavité de la matrice, soit dans le canal de l'utérus où ils engendrent une transformation de la glaire cervicale la rendant imperméable aux spermatozoïdes, ces divers modes d'action résultant du choix de l'emplacement de fixation du dispositif d'accrochage au tissu de la matrice. Le dispositif de l'invention constitue également un instrument tout à fait efficace dans le traitement des ménorragies, ainsi que dans te traitement des troubles de la ménopause, par libération topique de progestérone, en compensation des effets qui pourraient être néfastes pour l'utérus d'une thérapie systémique de substitution d'oestrogènes. Dans ce dernier cas également, une répartition aussi uniforme que possible de la progestérone libérée dans la cavité de la matrice est un élément important d'une application locale convenable.

**Revendications**

1. Dispositif intra-utérin constitué d'un élément déformable en brin ou en boucle, en une matière active dans la cavité de la matrice, solidaire d'un élément de fixation à la paroi de la matrice constitué d'un fil pourvu d'un dispositif d'accrochage au tissu de la matrice, convenant pour l'insertion à l'aide d'une aiguille, caractérisé en ce que l'élément déformable est une fibre (1;21;31;41) en matière plastique libérant une matière active dans la cavité de la matrice, en ce que cette fibre est formée en plusieurs brins ou boucles (2,3,3';23,23';32,32'; 42,42',42") maintenus ensemble en une même zone d'assemblage (4;24;34;44) de dimensions réduites par rapport à la longueur des brins ou des boucles.

2. Dispositif suivant 1, caractérisé en ce que le fil (5;25;35) pourvu d'un dispositif d'accrochage (6;26;36) au tissu de la matrice est rendu solidaire de la fibre (1;21;31) dans la zone d'assemblage (4;24;34) de cette dernière.

3. Dispositif suivant 1 et 2, caractérisé en ce que la fibre (1) est enroulée pour former au moins une boucle (2) et deux brins (3,3') de longueur approximativement égale à celle de la boucle, et en ce que l'assemblage de la boucle et des brins est réalisé par un noeud (4) formé dans le fil (5) pourvu du dispositif d'accrochage (6) au tissu de la matrice, ce noeud réalisant ainsi à la fois l'assemblage des divers éléments de la fibre et la fixation de la fibre au fil pourvu du dispositif d'accrochage au tissu de la matrice.

4. Dispositif suivant 1 ou 2, caractérisé en ce que l'élément d'assemblage est un élément d'assemblage (24) en forme d'anneau, libérant un espace intérieur (27) suffisant pour permettre le passage d'une aiguille.

5. Dispositif suivant 1, caractérisé en ce que l'élément d'assemblage est un point de colle (34;44)

**Claims**

1. An intra-uterine device consisting of a deformable element in the form of a strand or loop, made of a substance which is active in the uterine cavity, integral with a means for securing it to the wall of the uterus, which is a filament provided with a means for hooking to the tissue of the uterus, suitable for insertion with the aid of a needle, characterized in that the deformable element is a fibre (1; 21; 31; 41) made of plastic and releasing a substance which is active into the uterine cavity, and in that this fibre consists of a plurality of strands or loops (2, 3, 3'; 23, 23'; 32, 32'; 42, 42', 42") held together in one assembly zone (4; 24; 34; 44) of reduced dimensions in relation to the length of the strands or loops;

2. A device according to claim 1, characterized in that the filament (5; 25; 35) equipped with a means for hooking (6; 26; 36) to the tissue of the uterus, is rendered integral with the fibre (1; 21; 31) in the assembly zone (4; 24; 34) of the latter.

3. A device according to claim 1 and 2, characterized in that the fibre (1) is coiled to form at least one loop (2) and two strands (3, 3') the length of which is approximately equal to that of the loop; and in that the loop and strands are assembled with a knot (4) formed in the filament (5) provided with a means (6) for hooking to the tissue of the uterus, the said knot thus serving simultaneously to assemble the elements of the fibre and to secure the said fibre to the filament equipped with means for hooking to the tissue of the uterus.

4. A device according to claim 1 and 2, characterized in that the assembly element is in the form of ring (24) providing an internal space (27) sufficient to allow a needle to pass.

5. A device according to claim 1, characterized in that the assembly element is a spot of glue (34; 44).

**Patentansprüche**

1. Intra-uterine Vorrichtung, die aus einem verformbaren Faden- oder Schlingenelement aus einem in der Gebärmutterhöhle wirksamen Stoff besteht und fest mit einem Element zur Befestigung an der Gebärmutterwand verbunden ist, das einen Faden mit einer Vorrichtung zum Aufhängen am Gebärmuttergewebe aufweist, die zur Einführung mit Hilfe einer Nadel geeignet ist, dadurch gekennzeichnet, daß das verformbare Element eine Faser (1;21;31;41) aus Plastikmaterial ist, das einen in der Gebärmutterhöhle wirksamen Stoff freisetzt, daß die Faser aus mehreren Fäden oder Schlingen (2,3,3';23,23';32,32';42,42', 42") besteht, die in einem gemeinsamen Zusam-

EP 0 445 150 B1

menhaltestreifen (4;24;34;44) zusammengehalten werden, wobei die Ausdehnung hinsichtlich der Länge der Fäden oder Schlingen reduziert wird.

2. Vorrichtung nach Anspruch 1, dadurch gekennzeichnet, daß der mit einer Vorrichtung (6;26;36) zum Aufhängen am Gebärmuttergewebe ausgestattete Faden (5;25;35) fest mit der Faser (1;21;31) in deren Zusammenhaltestreifen (4;24;34) verbunden ist.

3. Vorrichtung nach Anspruch 1 und 2, dadurch gekennzeichnet, daß die Faser (1) zusammengerollt ist, um wenigstens eine Schlinge (2) und zwei Fäden (3,3') mit annähernd derselben Länge wie die Schlinge zu bilden und daß das Zusammenhalten der Schlinge und der Fäden durch einen Knoten (4) realisiert wird, der in dem mit einer Vorrichtung (6) zum Aufhängen am Gebärmuttergewebe ausgestatteten Faden (5) gemacht wird und so gleichzeitig die verschiedenen Elemente der Faser zusammenhält und die Faser an dem mit einer Vorrichtung zum Aufhängen am Gebärmuttergewebe ausgestatteten Faden befestigt.

4. Vorrichtung nach Anspruch 1 oder 2, dadurch gekennzeichnet, daß das Zusammenhalteelement ein Zusammenhalteelement (24) in Ringform ist und einen Innenraum (27) aufweist, der grob genug ist, um eine Nadel hindurchzuführen.

5. Vorrichtung nach Anspruch 1, dadurch gekennzeichnet, daß das Zusammenhalteelement ein Klebepunkt (34;44) ist.

FIG.1

FIG.2

FIG.3

FIG.4

6